# EUROPEAN PATENT APPLICATION

(11) **EP 4 019 013 A1**
(43) Date of publication of application: **29.06.2022**
(21) Application number: 20020654.8
(22) Date of filing: 24.12.2020
(51) Int. Cl.: A61K 31/05, A61P 11/00, A61K 31/12, A61K 31/198, A61K 31/519, A61K 45/06

(54) **TREATMENT OF PULMONARY DISORDERS**

(71) Applicant: Bionotus GCV, 9140 Temse (BE)
(72) Inventor: VAN Daele, Johan, 9140 Temse (BE); Annaert, Pieter, 9140 Temse (BE)
(74) Representative: Arnold & Siedsma

(57) **Abstract**

The present invention is related to the field of pulmonary disorders, more in particular to prevent, reverse or limit the progression of pulmonary disorders with an underlying vasoconstriction caused by disruption of the RAAS (reduced expression of the ACE2 receptor) and the kinin-kallikrein system (KKS), such as acute pulmonary vasoconstriction, traumatic injury, aspiration or inhalation injury, fat embolism in the lung, acidosis, inflammation of the lung, adult respiratory distress syndrome, acute pulmonary edema, acute mountain sickness, post cardiac surgery, acute pulmonary hypertension, persistent pulmonary hypertension of a newborn, perinatal aspiration syndrome, haline membrane disease, acute pulmonary thromboembolism, heparin-protamine reactions, sepsis, asthma, chronic pulmonary hypertension, bronchopulmonary dysplasia, chronic pulmonary thromboembolism and idiopathic or primary pulmonary hypertension or chronic hypoxia, and status asthmaticus or hypoxia.

## Description

### FIELD OF THE INVENTION

The present invention is related to the field of pulmonary disorders, more in particular to prevent, reverse or limit the progression of pulmonary disorders with an underlying vasoconstriction caused by disruption of the RAAS (reduced expression of the ACE2 receptor) and/or the kinin-kallikrein system (KKS) (hereinafter referred to as RAAS / KKS), such as acute pulmonary vasoconstriction, traumatic injury, aspiration or inhalation injury, fat embolism in the lung, acidosis, adult respiratory distress syndrome, acute pulmonary edema, acute mountain sickness, post cardiac surgery, acute pulmonary hypertension, persistent pulmonary hypertension of a newborn, perinatal aspiration syndrome, haline membrane disease, acute pulmonary thromboembolism, heparin-protamine reactions, sepsis, chronic pulmonary hypertension, bronchopulmonary dysplasia, chronic pulmonary thromboembolism and idiopathic or primary pulmonary hypertension or chronic hypoxia, and status asthmaticus or hypoxia.

### BACKGROUND TO THE INVENTION

Many pulmonary disorders are linked to an underlying constriction of the pulmonary blood vessels. In the majority of the pulmonary disorders this vasoconstriction of the pulmonary blood vessels is caused by disruption of the RAAS (reduced expression of the ACE2 receptor) and/or the kinin-kallikrein system (KKS). The symptoms associated with such disrupted RAAS / KKS system, include shortness of breath with minimal exertion, fatigue, dizzy spells, fainting and makes the lungs more sensitive to pulmonary embolism. If left untreated this may cause sequelae of haemodynamic changes that can even become life threatening.

A disruption of the RAAS / KKS system can have many causes, including the result of a viral respiratory infection, such as for example in case of common cold, influenza, severe acute respiratory syndrome (SARS) or COVID-19.

It is an object of the present invention to treat and prevent the problems that would otherwise be caused by the disruption of the RAAS / KKS system and accordingly prevent, reverse or limit the progression of the pulmonary disorders with an underlying vasoconstriction mentioned above.

### SUMMARY OF THE INVENTION

The present invention is based on the finding that resveratrol and other stilbenoids such as piceatannol derivable from amongst others *Abies picea* are able to significantly counteract on vasoconstriction caused by a disruption of the RAAS / KKS system by inducing the expression of Sirtuin proteins, in particular by activation of sirtuin 1 (SIRT1), a master regulator of Angiotensin Converting Enzyme 2 (ACE2) expression. Said activation of sirtuin 1 (SIRT1) by resveratrol and the resveratrol / stilbenoid analogues causes an increased ACE2 protein abundance and/or activity in the lung or in pulmonary tissue. This invention thus applies to any condition or disease that is characterized by a reduction of ACE2 abundance and/or activity, or any disease that may be treated by an induction of the ACE2 pool, or any condition or disease that might evolve into a situation where one could benefit from an increased ACE2 pool.

Resveratrol (3,4',5-trihydroxy-trans-stilbene) is a naturally occurring phenolic compound found, for example in grape skins. The term 'resveratrol' and 'resveratrol-containing composition' as used herein is meant to include both the 3,4',5-trihydroxy-trans-stilbene as well as the resveratrol / stilbenoid analogues such as the piceatannol analogue having the below structures; or combinations thereof.

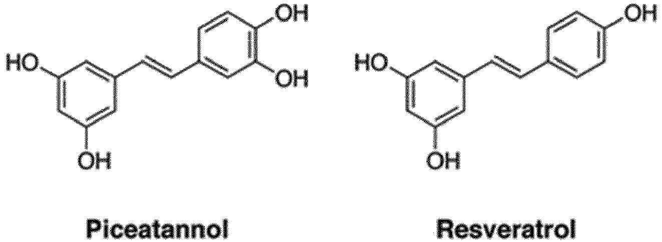

Like Resveratrol, piceatannol (3,3',4',5-trans-tetrahydroxystilbene) is a natural polyphenolic compound found in a limited number of plants, such as grapes, peanuts, Japanese knotweed, and some berries, and is particularly abundant in passion fruit (Passiflora edulis) seeds. Piceatannol is a 3'-hydroxylated analogue of resveratrol (3,4',5-trans-trihydroxystilbene). Both piceatannol and resveratrol have been reported to have anti-oxidative, anti-diabetic, antiinflammatory, and anti-cancer effects, wherein previous studies have shown that piceatannol is more potent than resveratrol in some aspects.

Based on the previously unrecognized benefit of resveratrol containing compositions in the treatment of pulmonary disorders, it is a first object of the present invention to provide such resveratrol-containing composition for use in the prevention or treatment of patients having a pulmonary disorder with pulmonary vasoconstriction and/or pulmonary embolism; more in particular in the treatment of pulmonary disorder with pulmonary vasoconstriction and/or pulmonary embolism, wherein said pulmonary vasoconstriction and/or pulmonary embolism is caused by a disruption of the RAAS / KKS system.

Acting through the activation of Sirtuin proteins, in particular by activation of sirtuin 1 (SIRT1), the resveratrol containing compositions can equally be used (both *in vitro* or *in vivo)* to increase Sirtuin protein activity; in particular SIRT1 activityin an epithelial cell; more in particular in a mucosal epithelial cell.

Known conditions wherein the pulmonary vasoconstriction and/or pulmonary embolism is caused by a disruption of the RAAS / KKS system include acute pulmonary vasoconstriction, traumatic injury, aspiration or inhalation injury, fat embolism in the lung, acidosis, adult respiratory distress syndrome, acute pulmonary edema, acute mountain sickness, post cardiac surgery, acute pulmonary hypertension, persistent pulmonary hypertension of a newborn, perinatal aspiration syndrome, haline membrane disease, acute pulmonary thromboembolism, heparin-protamine reactions, sepsis, chronic pulmonary hypertension, bronchopulmonary dysplasia, chronic pulmonary thromboembolism and idiopathic or primary pulmonary hypertension or chronic hypoxia, and status asthmaticus or hypoxia.

It is accordingly an embodiment of the present invention to provide a resveratrol containing composition for use in the prevention and/or treatment of a pulmonary condition selected from the group consisting of acute pulmonary vasoconstriction, traumatic injury, aspiration or inhalation injury, fat embolism in the lung, acidosis, adult respiratory distress syndrome, acute pulmonary edema, acute mountain sickness, post cardiac surgery, acute pulmonary hypertension, persistent pulmonary hypertension of a newborn, perinatal aspiration syndrome, haline membrane disease, acute pulmonary thromboembolism, heparin-protamine reactions, sepsis, chronic pulmonary hypertension, bronchopulmonary dysplasia, chronic pulmonary thromboembolism and idiopathic or primary pulmonary hypertension or chronic hypoxia, and status asthmaticus or hypoxia.

Using a symptomatic classification instead, the resveratrol-containing composition would be beneficial in the treatment of pulmonary disorders wherein the patients are selected from patients with shortness of breath symptoms; patients with diabetic phenotype; patients with hypertension; patients with lung injury such as for example due to chronic smoking, due to exposure to polluted air, due to irradiation, and due to viral infection; patients with metabolic syndrome; overweight and obese patients, and the like.

As will be further detailed herein below, the resveratrol-containing compositions are in first instance directed to compositions comprising resveratrol as the active pharmaceutical ingredient, wherein said resveratrol is present in an effective amount in a dosage form of the composition, i.e. in an amount that has a positive effect on vasoconstriction caused by a disruption of the RAAS / KKS system in a patient. This positive effect of resveratrol can be enhanced when used in combination with a further antioxidant; in particular an antioxidant having an immune enhancing effect such as N-acetylcysteine, curcumin and the like. It is accordingly an object of the invention to provide a resveratrol-containing composition for use in the treatment of the pulmonary disorders according to the invention, wherein said resveratrol-containing composition is further characterized in comprising an antioxidant; in particular in comprising N-acetylcysteine, curcumin or combinations thereof. Besides antioxidants also further active agents selected from anti-microbial agents, anti-viral agents, essential oils, monoterpenes, antihypertensives, vasodilators (such as sildefanil), corticosteroids, immunosuppressive and immunomodulatory compounds (such as beta-glucane), statins and bronchodilators could be present in the resveratrol-containing compositions for the applications as herein provided.

From the present invention being directed to the application of a resveratrol-containing composition in pulmonary disorders, it follows that in a preferred embodiment said resveratrol-containing composition consists a suitable dosage form that bypasses the stomach and the associated first pass effect of the liver which can remove up to 90% of the active ingested dose. Such dosage forms include administering the resveratrol-containing composition to the pulmonary, nasal, sublingual or buccal mucosae, typically by vaporisation or nebulisation, enemas or solid dosage forms such as gelsolets, gels, capsules, tablets, pastilles and lozenges.

Thus in a particular embodiment the resveratrol-containing compositions as herein provided are compositions for pulmonary, nasal, sublingual or buccal administration to the patients having a pulmonary disorder with pulmonary vasoconstriction and/or pulmonary embolism, or with an increased risk thereof; in particular a dry powder or aerosol composition for pulmonary and/or bucco-pharyngeal administration.

It is equally on object of the present invention to provide a method for the prevention or treatment of patients having a pulmonary disorder with pulmonary vasoconstriction and/or pulmonary embolism, said method comprising administering to said patients an effective amount of a resveratrol-containing composition; in particular in the treatment of patients wherein the pulmonary disorder with pulmonary vasoconstriction and/or pulmonary embolism is caused by a disruption of the RAAS / KKS system.

As for the composition for use above, also in the method for the prevention or treatment of the pulmonary disease indications wherein the resveratrol-containing compositions according to the invention are useful, are selected from acute pulmonary vasoconstriction, traumatic injury, aspiration or inhalation injury, fat embolism in the lung, acidosis, adult respiratory distress syndrome, acute pulmonary edema, acute mountain sickness, post cardiac surgery, acute pulmonary hypertension, persistent pulmonary hypertension of a newborn, perinatal aspiration syndrome, haline membrane disease, acute pulmonary thromboembolism, heparin-protamine reactions, sepsis, chronic pulmonary hypertension, bronchopulmonary dysplasia, chronic pulmonary thromboembolism and idiopathic or primary pulmonary hypertension or chronic hypoxia, and status asthmaticus or hypoxia.

Also here the patients that would benefit from a treatment with a resveratrol-containing composition could be identified symptomatically and are selected from patients with shortness of breath symptoms; patients with diabetic phenotype; patients with hypertension; patients with lung injury due to chronic smoking; patients with lung injury due to exposure to polluted air; patients with lung injury due to viral infection; patients with metabolic syndrome; overweight and obese patients.

In analogy with the compositions for use, also in the method of prevention or treatment the resveratrol-containing composition preferably further comprises an antioxidant, more in particular N-acetylcysteine; and is administered in a form that bypasses the stomach and the associated first pass effect of the liver; such as a pulmonary mucosa, nasal mucosa, sublingual mucosa or buccal mucosa route of administration.

### DETAILED DESCRIPTION OF THE INVENTION

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

In this specification and in the claims which follow, reference will be made to a number of terms and abbreviations which shall have their conventional meaning within the chemical and biological arts, unless indicated otherwise.

Resveratrol, a non-flavonoid polyphenolic antioxidant *(supra),* is one of the widely studied phytochemicals with demonstrated health potential due to its antioxidant, anticancer, and antiinflammatory properties, including inflammatory respiratory disorders such as disclosed in US 6,878,751. Interest in resveratrol has been renewed in recent years, first from its identification as a chemo preventive agent for skin cancer, and subsequently from reports that it activates sirtuin deacetylases and extends the lifespan of lower organisms, with a growing body of in vivo evidence which indicates that resveratrol has protective effects in rodent models of stress and disease.

Resveratrol as used herein may be synthesized chemically (Farina, A. et al. (2006) "An Improved Synthesis Of Resveratrol," Nat. Prod. Res. 20(3):247-252), or, more preferably, may be extracted from plant sources. Suitable sources of resveratrol include, but are not limited to resveratrol derived from natural sources such as grape skins, wine, or other botanical sources such as *P. cuspidatum* or *C. quinquangulata,* or produced synthetically such as 98% trans-resveratrol, available commercially from Sigma Chemical Co., St. Louis. Mo. Resveratrol for use in the compositions can be trans-resveratrol, cis-resveratrol, or a combination thereof. Botanical extracts with higher concentrations of resveratrol may be produced by fractionation and further column chromatography until an extract containing up to a 99% concentration of resveratrol (e.g. as a mixture of both cis- and trans-resveratrol) is produced. The compositions of the invention may also be made using modified resveratrol or derivatives of resveratrol. Some non-limiting examples of resveratrol's for making the compositions of the invention include, but are not limited to piceatannol *(supra)* and those disclosed in EP2774915, directed to derivatives of resveratrol which have favourable physicochemical properties when compared with resveratrol itself and are represented by formula (I) in said specification, the disclosures of which is incorporated by reference in its entirety for all purposes.

All stereoisomers (for example, geometric isomers, optical isomers and the like) of the present compounds (including those of the salts, solvates and complexes of the compounds), such as those which may exist due to asymmetric carbons on various substituents, including enantiomeric forms (which may exist even in the absence of asymmetric carbons), rotameric forms, atropisomers, and diastereomeric forms, are contemplated within the scope of this invention. Individual stereoisomers of the compounds of the invention may, for example, be substantially free of other isomers, or may be admixed, for example, as racemates or with all other, or other selected, stereoisomers. Generally, the active agent will be cis-resveratrol, trans-resveratrol, or a complex in which one or more of the compounds' hydroxyl groups are conjugated to for example a mono- or di-saccharide, e.g., cis-resveratrol glucoside, trans-resveratrol glucoside, etc.

The resveratrol's as provided herein may be administered in the form of a pharmacologically acceptable salt, ester, amide, prodrug or analog or as a combination thereof. Salts, esters, amides, prodrugs and analogs of resveratrol may be prepared using standard procedures known to those skilled in the art of synthetic organic chemistry and pharmaceutical formulation, described, for example, by J. March, "Advanced Organic Chemistry: Reactions, Mechanisms and Structure," 4th Ed. (New York: Wiley-Interscience, 1992), and in Remington's Pharmaceutical Sciences, 19th Ed. (Easton, Pa.: Mack Publishing Company, 1995). For example, basic addition salts are prepared from the neutral drug using conventional means, involving reaction of one or more of the active agent's free hydroxyl groups with a suitable base. Generally, the neutral form of the drug is dissolved in a polar organic solvent such as methanol or ethanol and the base is added thereto. The resulting salt either precipitates or may be brought out of solution by addition of a less polar solvent. Suitable bases for forming basic addition salts include, but are not limited to, inorganic bases such as sodium hydroxide, potassium hydroxide, ammonium hydroxide, calcium hydroxide, trimethylamine, or the like. Preparation of esters involves functionalization of hydroxyl groups which may be present within the molecular structure of the drug. The esters are typically acyl-substituted derivatives of free alcohol groups, i.e., moieties which are derived from carboxylic acids of the formula RCOOH where R is alkyl, and preferably is lower alkyl. Esters can be reconverted to the free acids, if desired, by using conventional hydrogenolysis or hydrolysis procedures. Preparation of amides and prodrugs can be carried out in an analogous manner. Other derivatives and analogs of the active agents may be prepared using standard techniques known to those skilled in the art of synthetic organic chemistry, or may be deduced by reference to the pertinent literature.

As mentioned herein before, the present invention is based on the finding that resveratrol is useful in the treatment of pulmonary disorders with pulmonary vasoconstriction and/or pulmonary embolism vessels caused by disruption of the RAAS (renin-angiotension-aldosterone system) and/or the kinin-kallikrein system (KKS) by reduced expression of the ACE2 receptor. The term "patient" as in treatment of "a patient" refers to a mammalian individual afflicted with or prone to a condition, disease or disorder as specified herein, and includes both humans and animals. The terms "treating" and "treatment" as used herein refer to reduction in severity and/or frequency of symptoms, elimination of symptoms and/or underlying cause, prevention of the occurrence of symptoms and/or their underlying cause, and improvement or remediation of damage. The term "pulmonary" as used herein refers to any part, tissue or organ that is directly or indirectly involved with gas exchange, i.e., O2/CO2 exchange, within a patient. "Pulmonary" contemplates both the upper and lower airway passages and includes, for example, the mouth, nose, pharynx, oropharynx, laryngopharynx, larynx, trachea, carina, bronchi, bronchioles and alveoli. Thus, the phrase "pulmonary drug administration" refers to administering the formulation described herein to any part, tissue or organ that is directly or indirectly involved with gas exchange within a patient.

The renin-angiotension-aldosterone system (RAAS) also referred to as renin-angiotensin system (RAS) generates, maintains, and makes worse hypertension and pulmonary vasoconstriction through its biologically active component angiotensin II (Ang II), that causes vasoconstriction, sodium retention, and structural alterations of the arteries. A few endogenous vasodilators, kinins, natriuretic peptides, and possibly angiotensin, exert opposite actions and may provide useful therapeutic agents. As endothelial autacoids, the kinins are potent vasodilators, active natriuretics, and protectors of the endothelium. Indeed, the kallikrein-kinin system (KKS) is considered the dominant mechanism for counteracting the detrimental effects of the hyperactive RAS. The 2 systems, RAS and KKS, are controlled by the angiotensin-converting enzyme (ACE) that generates Ang II and inactivates the kinins. Inhibitors of ACE can reduce the impact of Ang II and potentiate the kinins, thus contributing to restore the vascular homeostasis. ACE2 is responsible for Ang II hydrolysis, thus inhibiting RAS activity. Systemic decrease in ACE2 function impacts the Renin-Angiotensin-Kallikrein-Kinin systems (RAS-KKS) resulting in acute lung injury with increased vasoconstriction and associated embolisms/hypoxia.

Accordingly, the invention encompasses pharmaceutical formulations comprising resveratrol or an analogue thereof in association with a pharmaceutical carrier or diluent for use in the pulmonary disorders with pulmonary vasoconstriction and/or pulmonary embolism vessels caused by disruption of the RAAS (renin-angiotension-aldosterone system) and the kinin-kallikrein system (KKS) by reduced expression of the ACE2 receptor. The resveratrol-containing compositions may be formulated for administration by any route, for example oral, topical or parenteral. The compositions may, for example, be made up in the form of tablets, capsules, powders, granules, lozenges, creams, syrups, sprays or liquid preparations, for example solutions or suspensions, which may be formulated for oral use or in sterile form for parenteral administration by injection or infusion.

More generally, the compounds and compositions according to the invention may be formulated for administration in any convenient way for use in human or veterinary medicine.

Tablets and capsules for oral administration may be in unit dosage form, and may contain conventional excipients including, for example, binding agents, for example, syrup, acacia, gelatin, sorbitol, tragacanth, or polyvinylpyrrolidone; fillers, for example lactose, sugar, maize-starch, calcium phosphate, sorbitol or glycine; tableting lubricants, for example magnesium stearate, talc, polyethylene glycol or silica; disintegrants, for example potato starch; and pharmaceutically acceptable wetting agents, for example sodium lauryl sulphate. The tablets may be coated according to methods well known in normal pharmaceutical practice.

Oral liquid preparations may be in the form of, for example, aqueous or oily suspensions, solutions, emulsions, syrups or elixirs, or may be presented as a dry product for reconstitution with water or another suitable vehicle before use. Such liquid preparations may contain conventional additives, including, for example, suspending agents, for example sorbitol, methyl cellulose, glucose syrup, gelatin, hydroxyethyl cellulose, carboxymethyl cellulose, aluminium stearate gel or hydrogenated edible fats; emulsifying agents, for example lecithin, sorbitan monooleate or acacia; non-aqueous vehicles (which may include edible oils), for example almond oil, oily esters (for example glycerine), propylene glycol, or ethyl alcohol; preservatives, for example methyl or propyl p-hydroxybenzoate or sorbic acid; and, if desired, conventional flavouring and colouring agents.

In particular embodiments, the composition or mixture comprising the compounds of the invention may be realized in the form that bypasses the stomach and the associated first pass effect of the liver which can remove up to 90% of the active ingested dose. Such dosage forms include administering the resveratrol-containing composition to the pulmonary, nasal, sublingual or buccal mucosae, typically by vaporisation or nebulisation, enemas or solid dosage forms such as gels, capsules, tablets, pastilles and lozenges. When administered by nasal aerosol or inhalation, these compositions may be prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art. Suitable pharmaceutical formulations for administration in the form of aerosols or sprays are, for example, solutions, suspensions or emulsions of the compounds of the invention or their physiologically tolerable salts in a pharmaceutically acceptable solvent, such as ethanol or water, or a mixture of such solvents. If required, the formulation can also additionally contain other pharmaceutical auxiliaries such as surfactants, emulsifiers and stabilizers as well as a propellant. Dry powder formulations for pulmonary delivery include the active agent and any carrier suitable for pulmonary drug administration may be used, although pharmaceutical sugars are generally preferred as carriers, e.g., fructose, galactose, glucose, lactitol, lactose, maltitol, maltose, mannitol, melezitose, myoinositol, palatinite, raffinose, stachyose, sucrose, trehalose, xylitol, cyclodextrin, hydrates thereof and combinations thereof. Selected components are initially combined and then blended to form a homogeneous, uniform powder mixture. Techniques for preparation of such powders are well known in the art. Regardless of technique employed the resulting powder must be both homogeneous and uniform. Typically, the active agents will make up from about 0.10% to about 99% (w/w) of the total formulation. Liquid pulmonary formulations can also be used and are well known in the art. See, e.g., Remington: The Science and Practice of Pharmacy, supra. Preferably, the liquid is an aqueous suspension, but aqueous solutions may also be used. The liquid formulations include one or more carriers in addition to the active agents. Generally the carrier is a sodium chloride solution having concentration making the formulation isotonic relative to normal body fluid. In addition to the carrier, the liquid formulations may contain water and/or excipients including an antimicrobial preservative (e.g., benzalkonium chloride, benzethonium chloride, chlorobutanol, phenylethyl alcohol, thimerosal and combinations thereof), a buffering agent (e.g., citric acid, potassium metaphosphate, potassium phosphate, sodium acetate, sodium citrate, and combinations thereof), a surfactant (e.g., polysorbate 80, sodium lauryl sulfate, sorbitan monopalmitate and combinations thereof), and/or a suspending agent (e.g., agar, bentonite, microcrystalline cellulose, sodium carboxymethylcellulose, hydroxypropyl methylcellulose, tragacanth, veegum and combinations thereof). Combining the components followed by conventional mixing effects a liquid formulation suitable for inhalation. Typically, the active agents will make up from about 0.01% to about 40% of the total formulation.

Various known devices may be used to administer pulmonary formulations, whether dry powder, aerosol or liquid. Dry powder inhalers are well known to those skilled in the art and are used to administer the aforementioned dry powder formulations. Suitable dry powder inhalation devices for administering the present formulations include, for example, TURBOHALER^{®} (Astra Pharmaceutical Products, Inc., Westborough, Mass.), ROTAHALER^{®} (Allen & Hanburys, Ltd., London, England). Aerosol formulations may be administered via pressurized metered-dose inhalers, soft-mist inhalers and the like. Liquid formulations of the invention may be administered via a pump spray bottle or nebulizer.

Other active agents may also be included in the formulations of the invention, including other antioxidants, anti-microbial agents, anti-viral agents, essential oils, monoterpenes, antihypertensive, vasodilators (such as sildefanil), corticosteroids, statins, immonosuppresiva, immunomodulators and bronchodilators; in particular an antioxidants having an immune enhancing effect such as N-acetylcysteine, curcumin and combinations thereof.

The pharmaceutical preparations of the invention are preferably in a unit dosage form, and may be suitably packaged, for example in a box, blister, vial, bottle, sachet, ampoule or in any other suitable single-dose or multi-dose holder or container (which may be properly labeled); optionally with one or more leaflets containing product information and/or instructions for use. Generally, such unit dosages will contain between 0,01 and 500 mg, and usually between 0,05 and 100 mg, of the at least one compound of the invention, e.g. about 0,05, 0,1, 1, 5, 10, 20, 30, 40, 50, 60, 70, 80, 90 or 100 mg per unit dosage, wherein the unit dosage form when administered according to the appropriate dosing regimen will deliver an "effective amount", by which is meant any amount of the active agent that, upon suitable administration, is sufficient to achieve the desired therapeutic or prophylactic effect in the individual to which it is administered. Usually, depending on the condition to be prevented or treated and the route of administration, such an effective amount will usually be between 1 to 500 µg per kilogram body weight day of the patient per day, more often between 5 and 250 µg, such as between 1 and 100 µg, for example about 5, 10, 20, 30, 40, 50, 60, 70, 80, 90 or 100 µg, per kilogram body weight of the patient per day, which may be administered as a single daily dose, divided over one or more daily doses, or essentially continuously, e.g. using a drip infusion. The amount(s) to be administered, the route of administration and the further treatment regimen may be determined by the treating clinician, depending on factors such as the age, gender and general condition of the patient and the nature and severity of the disease/symptoms to be treated. In accordance with the method of the present invention, said pharmaceutical composition can be administered separately at different times during the course of therapy or concurrently in divided or single combination forms. The present invention is therefore to be understood as embracing all such regimes of simultaneous or alternating treatment and the term "administering" is to be interpreted accordingly.

The invention will now be illustrated by means of the following synthetic and biological examples, which do not limit the scope of the invention in any way.

### EXAMPLES

### In vitro exposure of human cell cultures to resveratrol or an analogue

Human primary cells and cell lines expressing Angiotensin Converting Enzyme-2 (ACE2), such as Caco-2 cells, Calu-3 cells, Vero cells, primary hepatocytes and HepG2 cells will be cultured *in vitro* according to standard cell culture conditions. After reaching confluence and/or after 24h-96h culture time in standard culture medium, cultures will be treated with resveratrol (or one of its analogues) at various predefined concentrations, between 0.1 and 500 µM. Parallel treatments with known sirtuin-1 (SIRT1) modulators will also be conducted as positive controls within the same experiment. Treatments will last for 0.1, 0.3, 0.5, 1, 3, 5, 12, 24, 48, 72 or 96h, and medium (containing resveratrol or an analogue) will be replaced every day (for treatments > 24h). Hanks Balanced Salt Solution (HBSS), containing 10 mM Hepes, pH 7.4 will be used for incubations of maximum 3h exposure time. For longer incubation, cell culture medium will be used. After these treatment period, cultures will be used immediately for determining activities and/or mRNA/protein expression as described below. Alternatively, a wash-out period of 12h-48h will be applied to distinguish between either: (i) direct effects of residual compound in the cells and, (ii) the indirect effect (up or down-regulation) of the treatment on mRNA and protein expression. For shorter incubation times (< 3h), the possibility to co-incubate resveratrol (or an analogue) and a fluorogenic substrate (see below) will be explored. All incubations will be performed in quintuplicate (five wells per condition and compound concentration). Results will be confirmed in at least one independent experiment (new batch of cells seeded).

### Determination of ACE2 activity in cell cultures

The fluorogenic peptide Mca-Tyr-Val-Ala-Asp-Ala-Pro-Lys(Dnp)-OH will be used as a substrate to determine ACE2 activity, both in medium samples and directly on cell cultures to which incubation buffer containing the substrate has been added. The optimal substrate concentration and incubation times will be determined in preliminary experiments. Samples from medium that was in contact with cell cultures during the treatment periods allows for the separate assessment of soluble ACE2 (activity), if present. Furthermore, incubation of the cells with freshly prepared buffer (HBSS with10 mM Hepes, pH 7.4) containing the fluorogenic peptide will allow to determine the activity of membrane bound ACE2. Both soluble and membrane-bound ACE2 activity will be determined after exposure of the cells to compounds of interest (e.g. resveratrol). The fluorogenic peptide substrate contains a highly fluorescent 7-methoxycoumarin group [Mca stands for (7-Methoxycoumarin-4-yl)acetyl] that is efficiently quenched by resonance energy transfer to the 2,4-dinitrophenyl (Dnp) group. It can be used to measure the activities of peptidases that are capable of cleaving an amide bond between the fluorescent group and the quencher group, causing an increase in fluorescence. It is an excellent substrate for caspase-1/interleukin-converting enzyme (ICE) and angiotensin I-converting enzyme-2 (ACE-2) (Enari, M. et al., 1996, Nature 380:723; Vickers, C. et al., 2002, J. Biol. Chem. 277:14838). The cleavage sites by ICE and ACE-2 are the peptide bonds between Ala and Asp and between Pro and Lys, respectively. If required, for instance to demonstrate specificity of the assay, determination of ACE2 activity will be repeated with endogenous RAS peptides followed by LC-MSMS analysis with validated methods.

### Determination of mRNA and/or protein expression of SIRT1, ACE2 and other genes of interest.

For determination mRNA levels, total RNA will be extracted from selected cultures and stored at -80°C until analysis with RNAseq in collaboration with a specialised genomics core facility. Based on a subsequent bioinformatics analysis, this will allow to identify transcriptome changes due to treatment with resveratrol or an analogue. The most pronounced changes at the mRNA level will be confirmed at the protein level, either by LC-MSMS based targeted proteomics or by Western blotting provided adequate antibodies can be obtained. It is expected that even acute exposure of cell cultures to resveratrol (or an analogue) will lead to increased membrane-bound ACE2 expression, due to activation of SIRT1.

## Claims

1. A resveratrol-containing composition for use in the preventive or curative treatment of patients having or at risk of a pulmonary disorder with pulmonary vasoconstriction and/or pulmonary embolism.

2. The resveratrol-containing composition for use according to claim 1, wherein the pulmonary disorder with pulmonary vasoconstriction and/or pulmonary embolism is caused by a disruption of the RAAS / KKS system.

3. The resveratrol-containing composition for use according to claim 1, wherein the pulmonary disorder with pulmonary vasoconstriction and/or pulmonary embolism is selected from acute pulmonary vasoconstriction, traumatic injury, irradiation, aspiration or inhalation injury, fat embolism in the lung, acidosis, inflammation of the lung, adult respiratory distress syndrome, acute pulmonary edema, acute mountain sickness, post cardiac surgery, acute pulmonary hypertension, persistent pulmonary hypertension of a newborn, perinatal aspiration syndrome, haline membrane disease, acute pulmonary thromboembolism, heparin-protamine reactions, sepsis, asthma, chronic pulmonary hypertension, bronchopulmonary dysplasia, chronic pulmonary thromboembolism and idiopathic or primary pulmonary hypertension or chronic hypoxia, and status asthmaticus or hypoxia.

4. The resveratrol-containing composition for use according to claim 1, wherein the patients are selected from patients with shortness of breath symptoms; patients with diabetic phenotype; patients with hypertension; patients with lung injury such as for example due to chronic smoking, due to exposure to polluted air, due to irradiation, and due to viral infection; patients with metabolic syndrome; overweight and obese patients, and the like..

5. The resveratrol-containing composition for use according to any one of claims 1 to 4, wherein the resveratrol-containing composition comprises further active agents selected from antioxidants, anti-microbial agents, anti-viral agents, essential oils, monoterpenes, antihypertensive, vasodilators (such as sildefanil), corticosteroids, statins, immono-suppresiva, immunomodulators and bronchodilators; in particular antioxidants having an immune enhancing effect such as N-acetylcysteine, curcumin; and combinations thereof.

6. The resveratrol-containing composition for use according to any one of claims 1 to 5, wherein the resveratrol-containing composition is a composition for pulmonary, nasal, sublingual or buccal administration to the patients having a pulmonary disorder with pulmonary vasoconstriction and/or pulmonary embolism.

7. The resveratrol-containing composition according to claim 6, wherein the resveratrol-containing composition is a dry powder composition for pulmonary and/or buccopharyngeal administration.

8. The resveratrol-containing composition according to claim 6, wherein the resveratrol-containing composition is an aerosol composition for pulmonary and/or buccopharyngeal administration.

9. A method of preventive or curative treatment of patients having or at risk of a pulmonary disorder with pulmonary vasoconstriction and/or pulmonary embolism, said method comprising administering to said patients an effective amount of a resveratrol-containing composition.

10. The method according to claim 9 wherein the pulmonary disorder with pulmonary vasoconstriction and/or pulmonary embolism is caused by a disruption of the RAAS / KKS system.

11. The method of claim 9, wherein the pulmonary disorder with pulmonary vasoconstriction and/or pulmonary embolism is selected from acute pulmonary vasoconstriction, traumatic injury, aspiration or inhalation injury, fat embolism in the lung, acidosis, inflammation of the lung, adult respiratory distress syndrome, acute pulmonary edema, acute mountain sickness, post cardiac surgery, acute pulmonary hypertension, persistent pulmonary hypertension of a newborn, perinatal aspiration syndrome, haline membrane disease, acute pulmonary thromboembolism, heparin-protamine reactions, sepsis, asthma, chronic pulmonary hypertension, bronchopulmonary dysplasia, chronic pulmonary thromboembolism and idiopathic or primary pulmonary hypertension or chronic hypoxia, and status asthmaticus or hypoxia.

12. The method of claim 9, wherein the patients are selected from patients with shortness of breath symptoms; patients with diabetic phenotype; patients with hypertension; patients with lung injury such as for example due to chronic smoking, due to exposure to polluted air, due to irradiation, and due to viral infection; patients with metabolic syndrome; overweight and obese patients, and the like.

13. The method of claim 9, wherein the resveratrol-containing composition comprises further active agents selected from antioxidants, anti-microbial agents, anti-viral agents, essential oils, monoterpenes, antihypertensive, vasodilators (such as sildefanil), corticosteroids, statins, immonosuppresiva, immunomodulators and bronchodilators; in particular an antioxidants having an immune enhancing effect such as N-acetylcysteine, curcumin; and combinations thereof.

14. The method of claim 9, wherein the effective amount of the resveratrol-containing composition is administered to the patient by a pulmonary, nasal, sublingual or buccal route of administration.
